# EUROPÄISCHE PATENTSCHRIFT

(11) **EP 3 025 683 B1**
(45) Veröffentlichungstag und Bekanntmachung des Hinweises auf die Patenterteilung: **25.12.2019**
(21) Anmeldenummer: 15191212.8
(22) Anmeldetag: 23.10.2015
(51) Int. Cl.: A61F 2/66, A61F 2/50

(54) **FUSSPROTHESE**
PROSTHETIC FOOT
PROTHESE DE PIED

(30) Priorität: 25.11.2014 DE 102014117210
(43) Veröffentlichungstag der Anmeldung: 01.06.2016
(73) Patentinhaber: Össur Iceland EHF, 110 Reykjavik (IS)
(72) Erfinder: KRANNER, Werner, 83043 Bad Aibling (DE); EPPENSTEINER, Max, 833737 Irschenberg (DE); NISSELS, Volker, 95445 Bayreuth (DE); HERTWIG, Jan, 83026 Rosenheim (DE)
(74) Vertreter: Klunker IP Patentanwälte PartG mbB

(56) Entgegenhaltungen:
- EP-A1- 2 420 212
- EP-A1- 2 644 167
- WO-A1-2007/085228
- DE-A1-102011 014 994
- US-A- 4 959 073

## Beschreibung

Die Erfindung betrifft eine Fußprothese.

Fußprothesen sind in unterschiedlicher Ausgestaltung bekannt, zum Beispiel aus der EP 2 420 212, die eine Fußprothese gemäß des Oberbegriffes von Anspruch 1 offenbart.

Ziel jeder Fußprothese ist es dem Träger ein möglichst komfortables und aufgrund der Federeigenschaften der Prothese gedämpftes Auftreten sowie ein dem realen Fuß angenähertes Laufen zu ermöglichen. Bekannt sind unter anderem Fußprothesen, die ein Unterteil aufweisen, das beim Laufen bodenseitig aufsetzt. Am Unterteil angeordnet ist eine elastisches Dämpfungselement, das primär Richtung Fersenbereich versetzt angeordnet ist. Auf diesem länglichen Dämpfungselement ist sodann eine Metallplatte mit einem geeigneten Befestigungsmittel, beispielsweise einer Pyramidenaufnahme vorgesehen, an welcher ein Verbindungsschaft oder dergleichen, mit dem die gesamte Prothese am Beinstumpf befestigt wird, angeordnet werden kann. Das Dämpfungselement stellt die elastische Beweglichkeit dieser Platte relativ zum Unterteil zur Verfügung. Um eine gewisse Vorspannung innerhalb des Dämpfungselements zu erreichen sind vor und hinter dem plattenseitigen Verbindungsmittel, also beispielsweise der Pyramidenaufnahme, Seil- oder Gurtschlaufen vorgesehen, die die Platte übergreifen und zum Unterteil laufen, und entweder an diesem befestigt sind oder dieses untergreifen. Hierüber wird die Halteplatte fest mit dem Unterteil verbunden respektive verspannt. Diese Seil- bzw. Gurtverbindungen sind insoweit flexibel, als ein Einfedern der Halteplatte relativ zum Unterteil möglich ist, und zwar sowohl im hinteren Fersenbereich als auch im vorderen, quasi fußmittig befindlichen Bereich. Jedoch wird ein Ausfedern und damit eine Relaxation des Dämpfungselements über die Seil- oder Gurtschlaufen begrenzt, so dass die Vorspannung bleibt. Aus dieser doppelten Bewegungsbegrenzung resultiert letztlich eine Begrenzung der Relativbeweglichkeit von Unterteil und Platte zueinander. Beim Aufsetzen mit dem Fersenabschnitt ist zwar ein fersenseitiges Einfedern möglich, jedoch kann das vordere Halteplattenende nicht folgen, da es über die dortigen Seil- oder Gurtschlaufe bewegungsbegrenzt ist. Beim Abrollen kann die Halteplatte zwar im vorderen Bereich relativ zum Unterteil einfedern, jedoch ist im Fersenbereich ein Ausfedern nicht möglich, da die Relativbeweglichkeit über die dortige Seil- oder Gurtverbindung begrenzt ist. Das heißt, dass eine Bewegung um den eigentlichen Drehpunkt zwischen dem Rückfuß und dem Vorderfußhebel, also zwischen Halteplatte und Unterteil, nur begrenzt möglich ist, da die Bewegung in beide Richtungen begrenzt ist.

Der Erfindung liegt damit das Problem zugrunde, eine demgegenüber verbesserte Fußprothese anzugeben.

Zur Lösung dieses Problems schlägt die Erfindung eine Fußprothese gemäß Anspruch 1 vor.

Erfindungsgemäß sieht die Fußprothese zum einen ein das Oberteil und das Unterteil bildendes einstückiges Bauteil vor, das bevorzugt aus einem Kohlenstofffaserverbundwerkstoff gefertigt ist, mithin also ein mehrlagiges Laminatbauteil ist. Oberteil und Unterteil sind über eine Umlenkung im Fußspitzenbereich miteinander verbunden, wobei die Umlenkung im Bereich zwischen z. B. 135° - 180° geführt ist. Am Oberteil befindet sich das Verbindungsmittel, also beispielsweise die Pyramidenaufnahme. Oberteil und Unterteil sind voneinander beabstandet, liegen jedoch näherungsweise deckungsgleich übereinander. Zwischen ihnen ist im Fersenbereich das elastische Dämpfungselement, beispielsweise aus einem gummiartigen Material, angeordnet, das wie beschrieben als Auftrittsdämpfer dient, mithin also die Relativbewegung von Oberteil zum Unterteil dämpft. Beide sind also über das Dämpfungselement miteinander gekoppelt, sie bilden im Bereich zwischen dem Dämpfungselement und der Fußspitze eine Feder, die beim Laufen gebogen und beansprucht wird. Das Dämpfungselement ist üblicherweise flächig mit dem Ober- und/oder Unterteil verbunden, zumeist verklebt.

Erfindungsgemäß ist nun ein druckweiches und zugfestes Verbindungsmittel vorgesehen, das das Oberteil und das Unterteil miteinander verbindet, wobei dieses Verbindungsmittel unmittelbar durch das Dämpfungselement in einer dortigen Ausnehmung geführt ist. Das heißt, dass das Dämpfungsmittel letztlich durch den Drehpunkt, gebildet von dem Dämpfungselement, geführt ist. Da das Dämpfungsmittel im fersennahen Bereich ist, in dem letztlich auch beispielsweise die Pyramidenaufnahme angeordnet ist, ist folglich die Verbindung des Oberteils mit dem Unterteil ebenfalls im Bereich unterhalb der Pyramidenaufnahme vorgesehen. Dieses Verbindungsmittel ist einerseits druckweich, das heißt, dass es bei Belastung nachgibt, so dass das Dämpfungselement einfedern kann. Darüber hinaus ist es aber auch zugfest, das heißt, dass ein Ausfedern des Dämpfungselements über das Verbindungsmittel begrenzt ist. Das Verbindungsmittel definiert im unbelasteten Zustand den maximalen Abstand von Oberteil zu Unterteil in diesen Bereich.

Da erfindungsgemäß das Verbindungsmittel durch das Dämpfungselement und damit quasi durch den Drehpunkt geführt ist, ist über dieses Verbindungsmittel die Relativbewegung von Oberteil zu Unterteil und damit das Verschwenken um den Drehpunkt weder im Fersenbereich noch im davor liegenden Fußmittelbereich respektive Fußspitzenbereich begrenzt. Das heißt, dass das Dämpfungselement und folglich das Oberteil zum Unterteil quasi um den Drehpunkt relativ weit und unbegrenzt einfedern respektive verschwenken können, mithin also eine deutlich verbesserte Relativbewegung von Oberteil zu Unterteil möglich ist. Der Federweg wird folglich nicht begrenzt, solange die Federn nicht in Kontakt stehen, was beim Abrollen über die Fußspitze zum Ende der Bewegung erfolgt. Bei einem Federkontakt, wenn also das Oberteil gegen das Unterteil läuft, wandert der quasi virtuelle Drehpunkt aus dem Dämpfungselement nach vorne in den Kontaktpunkt des Oberteils zum Unterteil, und die Verbindung spannt das Oberteil gegen das Unterteil, was zu einem progressiven Kraftanstieg führt. Es ist somit eine beachtliche Relativbeweglichkeit von Oberteil zum Unterteil in Fußlängsrichtung gegeben, was insbesondere durch die Kopplung über das einzige Verbindungsmittel, das durch das Dämpfungselement läuft, erreicht wird. Zum anderen kann hierzu auch noch beitragen, dass das Dämpfungselement bevorzugt nicht mit dem Oberteil verbunden ist, so dass sich dieses im Extremfall in Fußlängsrichtung gesehen relativ zum Dämpfungselement bewegen kann. Ein Abheben davon ist über das zugfeste Verbindungsmittel ausgeschlossen.

Insgesamt wird mit der erfindungsgemäßen Fußprothese folglich ein wesentlich komfortableres und dem realen Fuß angenähertes Laufen ermöglicht. Die auftretenden Schubkräfte in Anterior-Posterior-Richtung werden nicht vollständig unterbunden, da keine die Bewegung begrenzenden Seil- und Gurtverbindungen, wie einleitend beschrieben, vorgesehen sind, so dass keine ruckartigen und für den Anwender spürbaren Unterbrechungen in der Abrollbewegung entstehen.

Das Verbindungsmittel selbst ist bevorzugt ein Seil oder ein Band, wobei das Seil oder Band entweder aus Kunststofffasern, die hochfest und abriebfest sind, oder aus Stahldraht bestehen kann.

Hinsichtlich der Ausgestaltung des Verbindungsmittels sowie seiner Befestigung sind unterschiedliche Alternativen denkbar. Gemäß einer ersten Erfindungsalternative kann das Seil oder Band eine Schlaufe bilden, wobei die Enden in einem gemeinsamen Halteelement fixiert sind, das am Unterteil befestigt ist, während die Schlaufe über ein sie durchgreifendes Schlaufenhalteelement am Oberteil befestigt ist. Bei dieser Ausgestaltung ist eine feste Verbindung des Verbindungsmittels nur am Unterteil gegeben, wo das Halteelement entsprechend befestigt ist, während die Schlaufe am Oberteil über ein entsprechendes Schlaufenhalteelement fixiert ist.

Dieses Schlaufenhaltelement ist bevorzugt ein Stift, der an der Oberseite des Oberteils aufliegt und der die das Oberteil in einer Durchbrechung durchgreifende Schlaufe durchgreift. Der Stift dient also als Wiederlager, an dem die Schlaufe eingehängt ist. Er ist seinerseits an der Oberseite des Oberteils aufgelagert, so dass folglich eine feste Verbindung von Oberteil zum Unterteil gegeben ist.

Zweckmäßig ist es, wenn am Oberteil ein Halteelement angeordnet ist, über das der Stift gegen ein axiales Verschieben gesichert ist. Über dieses Halteelement, das an der Oberseite des Oberteils angeordnet ist, ist der Stift folglich in seiner Position fixiert, er kann sich also nicht in seiner Längsrichtung bewegen und im Extremfall aus der Schlaufe herausrutschen. Das Halteelement ist beispielsweise plättchenförmig ausgeführt und kann eine oder zwei abstehende Laschen, die in die oberteilseitige Durchbrechung eingreifen, sowie zwei weitere Laschen, zwischen denen der Stift aufgenommen ist, aufweisen. Durch die plättchenartige Ausgestaltung baut das Halteelement in keinem Fall allzu hoch auf. Über die zu seiner Unterseite vorspringende eine oder über die beiden vorspringenden Laschen, die in die oberteilseitige Durchbrechung, durch die die Schlaufe läuft, eingreifen, ist das Halteelement quasi in Querrichtung respektive in Längsrichtung des an ihm fixierten Stiftes positionsfixiert, kann sich also selbst nicht in diese Richtung bewegen. Der Stift selbst ist zwischen zwei weiteren Laschen, die an der Oberseite des Halteelements abstehen, fixiert.

Eine solche Ausgestaltung des Halteelements ist insbesondere dann einfach herstellbar, wenn das Halteelement ein Halteblech ist. Denn die entsprechende Durchbrechung des Halteelements, durch die Schlaufe greift, sowie die Laschen können ohne weiteres in einem einfachen Stanzvorgang ausgeformt werden.

Alternativ dazu kann der Stift auch in eine an der Oberseite des Oberteils angeordnete Halteplatte eingesteckt sein. Diese Halteplatte kann beispielsweise bei entsprechender Ausgestaltung des Oberteils das Befestigungsmittel, also beispielsweise die Pyramidenaufnahme tragen. Da diese Halteplatte oberhalb des Dämpfungselements bei einer solchen Prothesenausgestaltung anzuordnen ist, bietet sich in diesem Fall die Fixierung des Stiftes an der Halteplatte an.

Die Durchbrechung, die von der Schlaufe durchgriffen ist, ist bevorzugt als Schlitz, der sich längs des Oberteils erstreckt, ausgeführt. Dieser Schlitz kann sich beispielsweise bis in den mittleren Prothesenbereich erstrecken, erweist also eine gewisse Länge auf. Er ermöglicht im Extremfall sogar eine gewisse Beweglichkeit der Schlaufe in dem Schlitz respektive des Stiftes, sofern dieser auf der Oberfläche direkt aufliegt. Wie beschrieben kommt es beim Abrollen zu einer Berührung von Oberteil und Unterteil und bei einem weiteren Abrollen zu einem hohen Kraftanstieg, verbunden mit einer Relativverschiebung des Oberteils zum Unterteil. Dieser kann die Schlaufe und der Stift wenn nötig letztlich folgen, da sie sich aufgrund der Schlitzausgestaltung geringfügig bewegen können. Alternativ zur Ausführung als Schlitz ist es selbstverständlich auch denkbar, die Durchbrechung als einfache Bohrung auszuführen.

Eine Erfindungsalternative zu der beschriebenen Ausgestaltung, bei der das Seil oder Band mittels eines Stiftes fixiert ist, sieht vor, das Seil oder Band mit beiden Enden jeweils in einem Halteelement zu fixieren, wobei das eine Halteelement am Unterteil und das andere Halteelement am Oberteil befestigt ist. Hier läuft also das Seil oder Band geradlinig vom Unterteil zum Oberteil, wo es über die Halteelemente entsprechend befestigt ist.

Ein solches Halteelement, unabhängig davon, ob nun eines oder zwei vorgesehen sind, ist bevorzugt als Hülse ausgeführt, in der das Seil oder Band eingeklebt, eingegossen oder eingeklemmt ist, wobei die Hülse das Unterteil oder das Oberteil in einer Durchbrechung durchgreift. Beispielsweise ist an der Hülse ein Radialbund vorgesehen, der am Unterteil oder am Oberteil, sofern auch dort eine solche Hülse zu befestigen ist, anliegt. Sowohl bei der Schlaufenausführung als auch bei der Ausführung ohne Schlaufe wird das Seil oder Band in der am Unterteil festzulegenden Hülse zunächst eingeklebt oder eingegossen oder dort entsprechend verklemmt, wobei bevorzugt das Eingießen oder Einkleben genutzt wird. Anschließend wird die Hülse am Unterteil eingesetzt und das Seil oder Band in die Durchbrechung am Dämpfungselement geführt. Die Hülse wird nun am Unterteil befestigt, beispielsweise ebenfalls verklebt oder eingegossen. Sodann wird das Oberteil fest gegen das Unterteil gedrückt, so dass das Dämpfungselement komprimiert wird. Dies ermöglicht es, den Stift durch die z. B. durch den Schlitz geführte Schlaufe zu schieben und entsprechend am Oberteil oder der Halteplatte zu positionieren, woraufhin der Druck wieder entlastet wird und das Verbindungsmittel montiert ist. Wird eine zweite Hülse am Oberteil benötigt, so wird das dortige Seilende in die Hülse eingesetzt und in dieser verklebt oder vergossen, wonach nach Aushärten des Vergusses oder des Klebers der Druck entlastet wird und die Hülse, beispielsweise ebenfalls über einen entsprechenden Radialbund, entsprechend am Oberteil fixiert ist. Die Hülse kann sich dabei natürlich jeweils ein Stück weit in das Elastomer erstrecken, das eine entsprechende Aufweitung in diesem Bereich aufweist.

Eine dritte Ausgestaltung hinsichtlich der Verbindung des Seils oder Bandes mit dem Oberteil und dem Unterteil sieht vor, das Seil oder Band unmittelbar am Oberteil und am Unterteil einzulaminieren. Wie beschrieben besteht das das Unterteil oder Oberteil bildende Bauteil bevorzugt aus einem Kohlenstofffaserverbundwerkstoff, der üblicherweise aus mehreren übereinander laminierten Kohlenstofffaserlagen besteht. Im Rahmen dieses Herstellungsprozesses ist es nun denkbar, sogleich das Seil oder Band mit seinen Fasern oder Drähten am Oberteil und am Unterteil einzulaminieren, so dass es letztlich ebenfalls Teil des einstückigen Bauteils ist. Es ist sodann lediglich noch erforderlich, das Dämpfungselement zu montieren, das zu diesem Zweck natürlich eine schlitzartige Öffnung aufweisen sollte, um es zwischen Oberteil und Unterteil zu schieben.

Wie beschrieben ist ein wesentliches Element, dass das Seil oder Band das Dämpfungselement in einer entsprechenden Ausnehmung durchsetzt. Diese Ausnehmung kann als Durchgangsbohrung ausgeführt sein, oder als Schlitz, der gegebenenfalls an einer Seite des Dämpfungselements offen ist. Ist das Dämpfungselement vor dem Montieren des Seiles oder Bandes bereits am Unterteil verklebt, so ist die Ausnehmung bevorzugt als Durchbrechung ausgeführt, durch die das Seil oder Band geführt werden kann. Wird das Seil oder Band zuvor montiert, so ist die Ausnehmung zweckmäßigerweise als Schlitz ausgeführt, so dass das Dämpfungselement zwischen Oberteil und Unterteil geschoben werden kann und dabei das Seil oder Band in den Schlitz läuft.

Zur Variation des Dämpfungsverhaltens kann am Dämpfungselement wenigstens ein dessen Dämpfungsverhalten beeinflussendes Verstärkungselement anbringbar sein. Hierzu ist beispielsweise eine Einsteckaufnahme am Dämpfungselement vorgesehen, in die das Verstärkungselement einsteckbar ist. Es kann sich hierbei um eine formschlüssige Verbindung handeln, das heißt, dass die Einsteckaufnahme wie auch das Verstärkungselement entsprechend geometrisch ausgeformt sind. Ein solches Verstärkungselement, z. B. aus einem eine andere Härte als das Dämpfungselement aufweisenden Kunststoff, kann beispielsweise nur an der Fersenrückseite des Dämpfungselements vorgesehen sein, oder auch an der gegenüberliegenden Seite.

Schließlich kann eine am Oberteil lösbar anbringbare Abdeckung vorgesehen sein, die den Befestigungsbereich des Verbindungsmittels abdeckt, wobei eine solche Abdeckung natürlich nur dann benötigt wird, wenn das Seil oder Band entweder über den Stift oder eine oberteilseitig festgelegte Hülse fixiert ist.

Weitere Vorteile und Einzelheiten der Erfindung ergeben sich aus dem im Folgenden beschriebenen Ausführungsbeispiel sowie anhand der Zeichnungen. Dabei zeigen:
- Figur 1: eine Perspektivansicht einer erfindungsgemäßen Fußprothese,
- Figur 2: eine Perspektivansicht um 90° gedreht,
- Figur 3: eine Seitenansicht der Fußprothese,
- Figur 4: eine Perspektivansicht des Dämpfungselements mit zugeordnetem Verbindungsmittel von schräg oben,
- Figur 5: die Anordnung aus Figur 4 von schräg unten,
- Figur 6: eine Vorderseitenansicht der Anordnung aus Figur 4,
- Figur 7: eine Schnittansicht durch die Anordnung in Richtung der Linie VII-VII aus Figur 6,
- Figur 8: eine Prinzipdarstellung dreier unterschiedlicher Laufsituationen zur Erläuterung der Relativbeweglichkeit von Oberteil zu Unterteil,
- Figur 9: eine weitere Prinzipdarstellung dazu,
- Figur 10: eine Schnittansicht durch eine Fußprothese einer zweiten Ausführungsform,
- Figur 11: eine Prinzipdarstellung einer weiteren Möglichkeit der Befestigung des Seiles oder Bandes,
- Figur 12: eine Prinzipdarstellung einer weiteren Möglichkeit der Befestigung des Seiles oder Bandes mit zwei Hülsen,
- Figur 13: eine Perspektivansicht eines Halteelements in Form eines Halteblechs,
- Figur 14: eine Perspektivdarstellung des Halteelements mit daran angeordnetem Stift, und
- Figur 15: eine Perspektivansicht einer erfindungsgemäßen Fußprothese mit Halteelement und Stift.

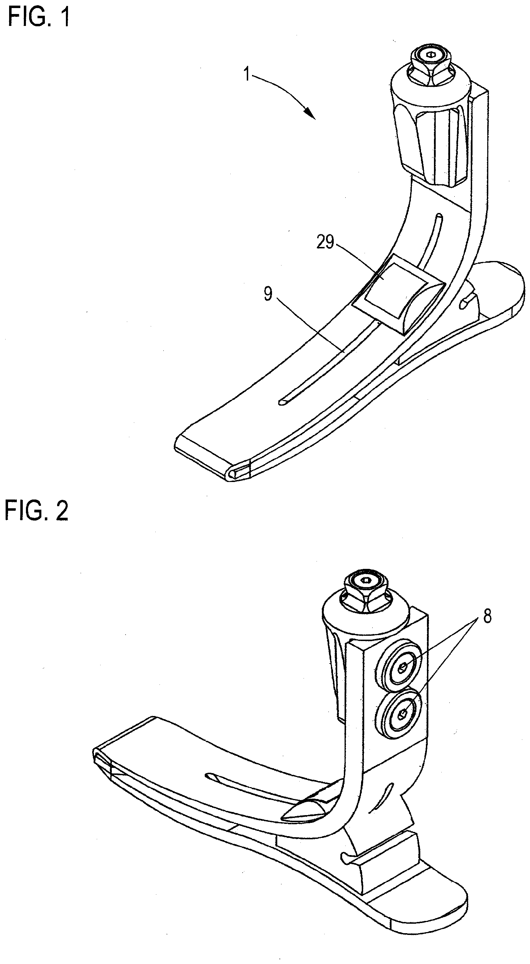

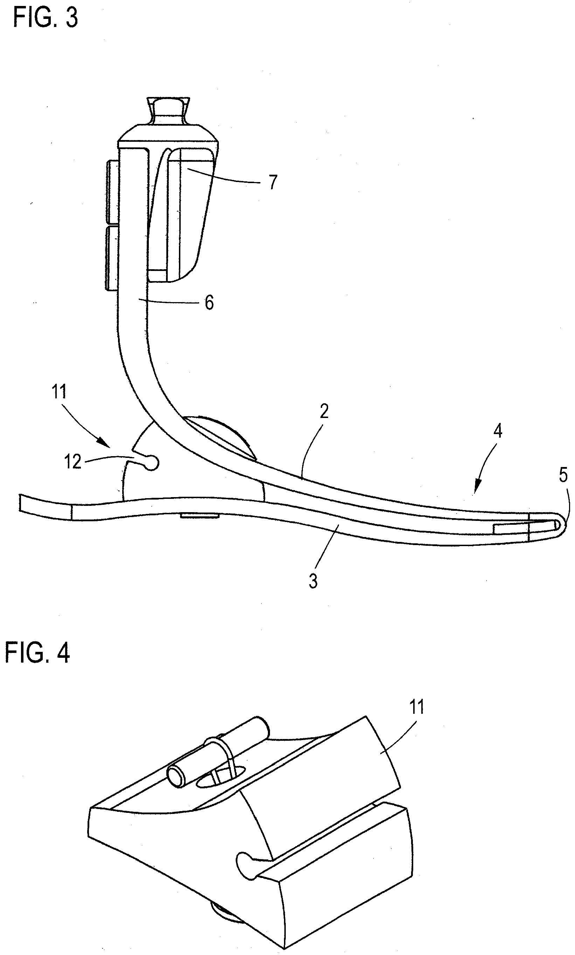

Figur 1 zeigt eine erfindungsgemäß Fußprothese 1, bestehend aus einem Oberteil 2 und einem Unterteil 3, siehe hierzu auch die Figuren 2 und 3. Das Oberteil 2 und das Unterteil 3 sind mittels eines einstückigen Bauteils 4 realisiert, wobei das Oberteil 2 und das Unterteil 3 im Fußspitzenbereich über eine Umlenkung 5 miteinander verbunden sind. Das Oberteil 2 läuft in diesem Ausführungsbeispiel in einen vertikalen Abschnitt 6 aus, an dem ein Befestigungsmittel 7 zur Verbindung beispielsweise eines Prothesenschaftes über geeignete Befestigungsschrauben 8 angeordnet ist.

Das Bauteil 4 ist ein einstückiges Bauteil, vorzugsweise aus einem Kohlenstofffaserverbundwerkstoff. Das heißt, dass das Oberteil 2 und das Unterteil 3 in einem einzigen Herstellvorgang ausgebildet werden können. Ersichtlich liegen das Oberteil 2 und das Unterteil 3 vertikal gesehen deckungsgleich übereinander, sie verlaufen also übereinander.

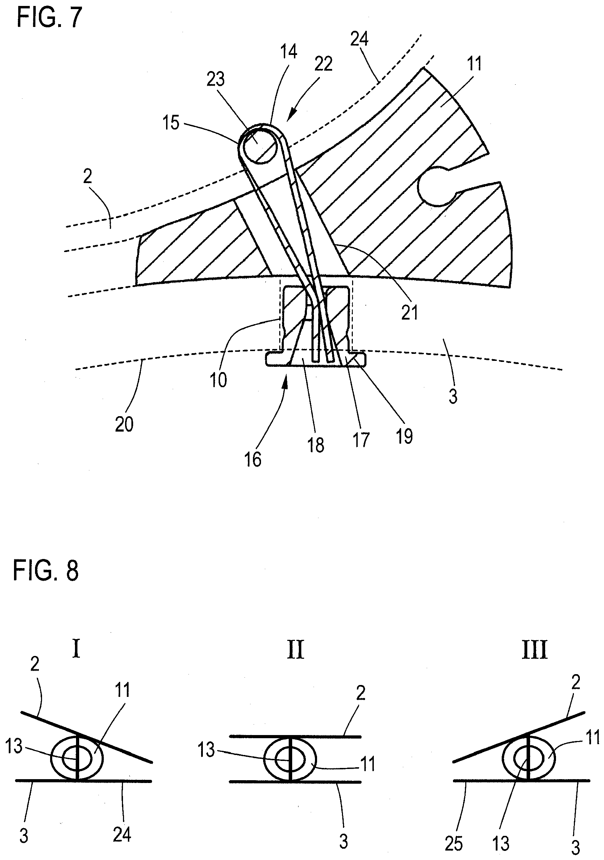

Am Oberteil 2 ist ein längslaufender Schlitz 9 vorgesehen, auf den nachfolgend noch eingegangen wird. Am Unterteil 3 ist, siehe Figur 7, eine Durchbrechung 10 ausgebildet, auf die nachfolgend ebenfalls noch eingegangen wird. Schlitz 9 und Durchbrechung 10 dienen der Anordnung eines Verbindungselements, das nachfolgend ebenfalls noch beschrieben wird.

Zwischen dem Oberteil 2 und dem Unterteil 3 ist ein Dämpfungselement 11 aus einem elastischen, gummiartigen Material angeordnet. Es ist vorzugsweise nur mit dem Unterteil 3 fest verbunden, beispielsweise verklebt. An der fersennahen Rückseite des Dämpfungselements 11 ist eine Einsteckaufnahme 12 ausgebildet, in die zur Variation des Dämpfungsverhaltens bei Bedarf ein Einsteckelement, das hier nicht näher gezeigt ist, eingesetzt werden kann, das dort formschlüssig gehaltert ist. Oberseitig ist eine Abdeckung 29 lösbar am Oberteil 2 angeordnet.

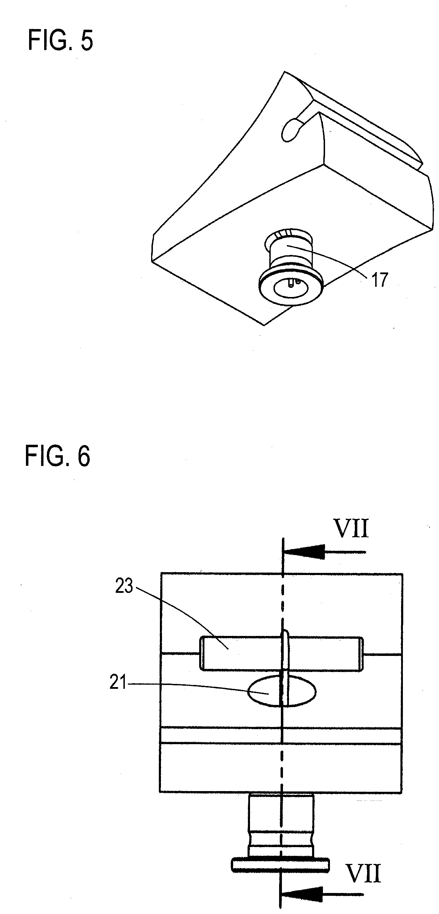

Die Figuren 4 - 7 zeigen im Detail das Dämpfungselement 11 sowie das es durchgreifende Verbindungsmittel 13, über das das Oberteil 2 und das Unterteil 3 miteinander verbunden sind. Das Dämpfungselement weist eine Durchbrechung 21 auf, die im gezeigten Beispiel als einfache Durchgangsbohrung ausgeführt ist, siehe insbesondere die Schnittansicht in Figur 7. Das Verbindungsmittel 13 umfasst in diesem Ausführungsbeispiel ein Seil 14, vorzugsweise ein Seil aus Kunststofffasern, denkbar ist aber auch die Verwendung eines Drahtseils. Das Seil 14 ist als Schlaufe 15 ausgeführt. Mit seinen beiden Enden ist das Seil 14 in einem Halteelement 16, hier in Form einer Hülse 17, aufgenommen und darin über eine Vergussmasse 18 fest verankert. In der Montagestellung ist die Hülse 17 in die Durchbrechung 10 am Unterteil eingesetzt und liegt über einen Radialbund 19 an der Unterseite 20 des Unterteils 3 auf.

Das Seil 14 ist durch die Durchbrechung 21 des Dämpfungselements 11 geführt und tritt zum Oberteil 2 hin aus. Es durchgreift das Oberteil 2 im Schlitz 9, ist also durch den Schlitz 9 durchgeführt. Ein Schlaufenhalteelement 22, hier in Form eines Stiftes 23, ist durch die Schlaufe 15 gesteckt. In der Montagestellung liegt der Stift 23, siehe die Schnittansicht gemäß Figur 7, auf der Oberseite 24 des Oberteils 2 auf. Hierüber erfolgt die Fixierung des Seils 14 am Oberteil 2.

Über das Verbindungsmittel 13 ist folglich eine druckweiche, jedoch zugfeste Verbindung von Oberteil und Unterteil realisiert, wobei sich das Verbindungsmittel 13 durch das Dämpfungselement 11 erstreckt. Da das Dämpfungselement 11 letztlich den Drehpunkt bildet, um den das Oberteil 2 relativ zum Unterteil 3 beim Laufen während des Aufsetzens der Ferse bis zum Abrollvorgang über die Fußspitze dreht, ist folglich keinerlei Bewegungsbegrenzung respektive Hebelbegrenzung im Rückfuß- und Vorderfußhebelbereich gegeben.

Die Prinzipdarstellungen gemäß Figur 8 machen dies deutlich. Die Figur 8 zeigt in Form von drei Prinzipdarstellungen I, II und III die Relativbewegung des Oberteils 2 zum Unterteil 3. Es sei angenommen, dass die Ansicht I den Fersenaufsetzvorgang, wenn also der Fersenabschnitt 24 des Unterteils 3 am Boden aufsetzt, zeigt. Ersichtlich kann hierbei das Oberteil 2 zur Ferse hin einschwenken respektive einfedern, ohne dass das Verbindungsmittel 13, also die Seilverbindung, diese Bewegung in irgendeiner Weise begrenzt. In Figur 8 ist der jeweilige Verschwenkbereich überzogen dargestellt, gleichwohl ist hieraus das Grundprinzip ersichtlich.

Darstellung II zeigt den Zustand, wenn die Prothese flächig mit dem Unterteil am Boden aufliegt. Oberteil 2 und Unterteil 3 laufen, lediglich vertikal belastet, übereinander, wobei der Übergang von der Situation gemäß Darstellung I zur Darstellung II über das Verbindungsmittel 13 in keiner Weise behindert ist.

Darstellung III zeigt schließlich den Abrollvorgang über die Fußspitze 25 des Unterteils 3. Hier kann das Oberteil 2 im Bereich der Fußspitze zum Unterteil 3 einfedern, während im Fersenbereich eine Auseinanderbewegung möglich ist.

Auch diese Bewegung ist in keiner Weise über das Verbindungsmittel 13 begrenzt oder eingeschränkt.

Damit gewährleistet die erfindungsgemäße Prothese, dass die auftretenden Schubkräfte in Anterior-Posterior-Richtung nicht vollständig unterbunden werden respektive es in keiner Weise zu einer durch das Verbindungsmittel begrenzten Relativbeweglichkeit kommt, so dass keine ruckartigen und für den Anwender spürbaren Unterbrechungen in der Abrollbewegung entstehen. Vielmehr ergibt sich ein runder, homogener Aufsetz- und Abrollvorgang.

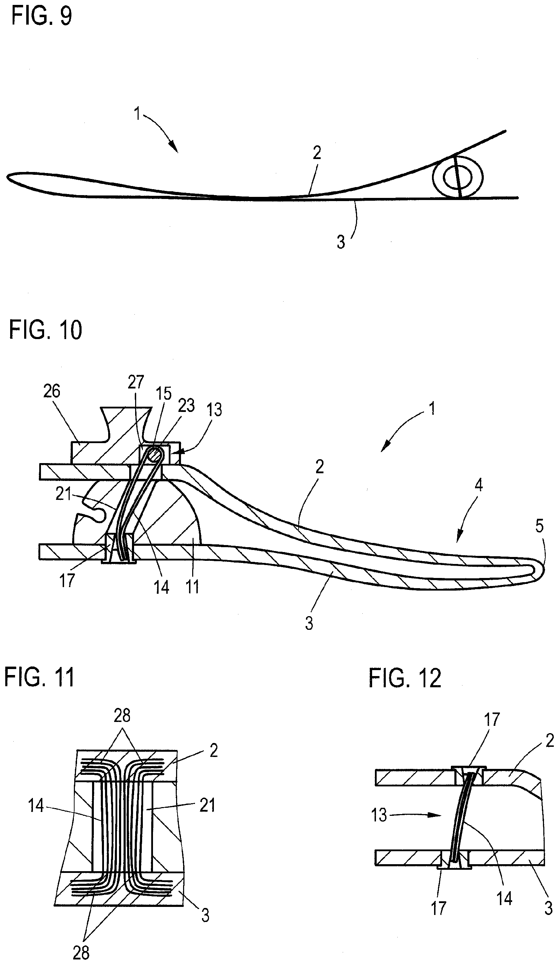

Figur 9 zeigt eine Prinzipdarstellung der gesamten Fußprothese 1, entsprechend den Prinzipdarstellungen aus Figur 8. Ersichtlich kommt es beim Laufen beim Übergang von Darstellung II zur Darstellung III zu einem Anliegen des Oberteils 2 am Unterteil 3. Bei diesem Kontakt wandert der virtuelle Drehpunkt aus dem Dämpfungselement 11 in den Kontaktpunkt zwischen Oberteil 2 und Unterteil 3. Die aus Oberteil 2 und Unterteil 3 gebildete Feder wird also gegeneinander gespannt, es kommt zu einem progressiven Kraftanstieg bei fortgesetzter Abrollbewegung.

Figur 10 zeigt eine weitere Ausgestaltung einer erfindungsgemäßen Fußprothese 1, wobei für gleiche Bauteile gleiche Bezugszeichen verwendet werden. Auch diese weist ein Oberteil 2 und ein Unterteil 3 auf, die aus einem einstückigen Kohlenstofffaserverbund-Bauteil 4 bestehen. Sie gehen auch hier über eine Umlenkung 5 ineinander über.

Vorgesehen ist wiederum ein Dämpfungselement 11 zwischen Oberteil 2 und Unterteil 3, wobei bei dieser Ausgestaltung das Oberteil 2 geometrisch gesehen anders ausgeführt ist als bei der zuvor beschriebenen Ausgestaltung. Hier läuft das Oberteil 2 zum Fersenbereich hin quasi genähert parallel zum Unterteil 3. Am Oberteil 2 ist eine Halteplatte 26 angeordnet, die das Befestigungselement trägt, an dem beispielsweise ein Verbindungsschaft angebracht wird.

Vorgesehen ist auch hier wiederum ein Verbindungsmittel 13, umfassend ein Seil 14, das eine Schlaufe 15 bildet. Die unteren Seilenden sind wiederum in einer Hülse 17 aufgenommen, die am Unterteil in entsprechender Weise wie zu den vorhergehenden Figuren beschrieben, befestigt ist. Das Seil 14 läuft wiederum durch eine Durchbrechung 21 im Dämpfungselement 11. Es ist durch eine im Oberteil 2 vorgesehene Durchbrechung zu einer Ausnehmung 27 in der Halteplatte 26 geführt, in welcher Ausnehmung ein Stift 23 eingesteckt ist, an dem wiederum die Schlaufe 15 eingehängt ist. Der Stift 23 ist in einer entsprechenden Einsteckhalterung an der Halteplatte 26 fixiert. Hier findet folglich die Befestigung des Stiftes 23 nicht unmittelbar am Oberteil 2, sondern an der oberteilseitig befestigten Halteplatte 26 statt.

Figur 11 zeigt eine weitere Ausgestaltung, bei der am Oberteil 2 und am Unterteil 3 das Seil 14 unmittelbar einlaminiert ist. In der Prinzipdarstellung sind die einzelnen Seilfasern 28 gezeigt, die im Oberteil 2 und am Unterteil 3 direkt in die Laminat- oder Harzmatrix eingebunden sind. Sie durchlaufen wiederum das Dämpfungselement 11, das bei dieser Ausgestaltung erst nachträglich montiert wird, und das hierzu beispielsweise einen geeigneten Schlitz aufweist, der in die entsprechende Durchbrechung 21 führt, so dass das Dämpfungselement 11 zwischen Oberteil 2 und Unterteil 3, das Seil 14 in sich aufnehmend, geschoben werden kann.

Figur 12 zeigt schließlich eine Ausführungsform, bei der das Verbindungsmittel 13 sowohl eine an dem Unterteil 3 festgelegte Hülse 17 als auch eine an dem Oberteil 2 festgelegte Hülse 17 aufweist. Das Seil 14 ist hier folglich mit seinen Enden in zwei Hülsen 17 fixiert, es bildet bei dieser Ausgestaltung keine Schlaufe. Die Funktion ist jedoch grundsätzlich die gleiche wie zu den vorstehenden Ausführungsformen beschrieben.

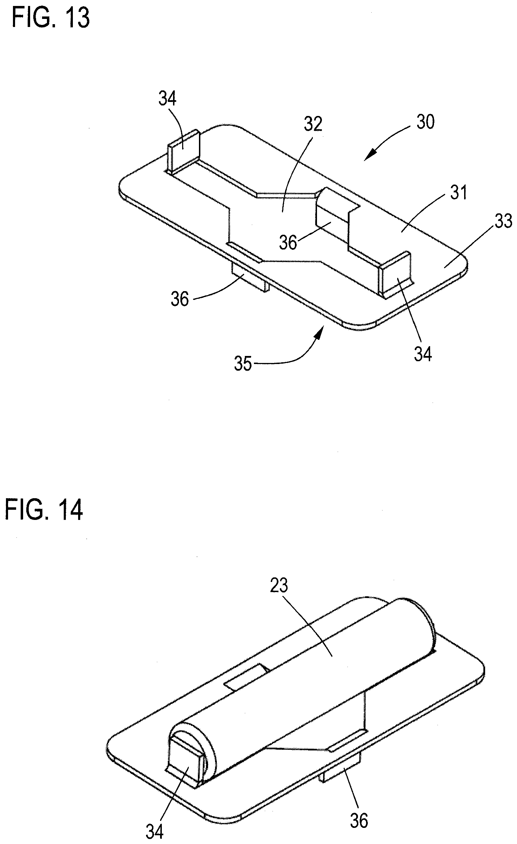

Figur 13 zeigt ein Halteelement 30 in Form eines plättchenförmigen Halteblechs 31, das der Fixierung des bei der Ausführungsform gemäß der Figuren 1 - 7 vorgesehenen Stiftes dient. Das Halteblech 31 weist eine mittige Durchbrechung 32 auf. An der Oberseite 33 sind zwei von ihr abstehende Laschen 34 vorgesehen, zwischen denen, siehe Figur 14, der Stift 23 aufgenommen ist. Hierüber ist er folglich axial festgelegt, er kann sich also nicht in seiner Längsrichtung bewegen.

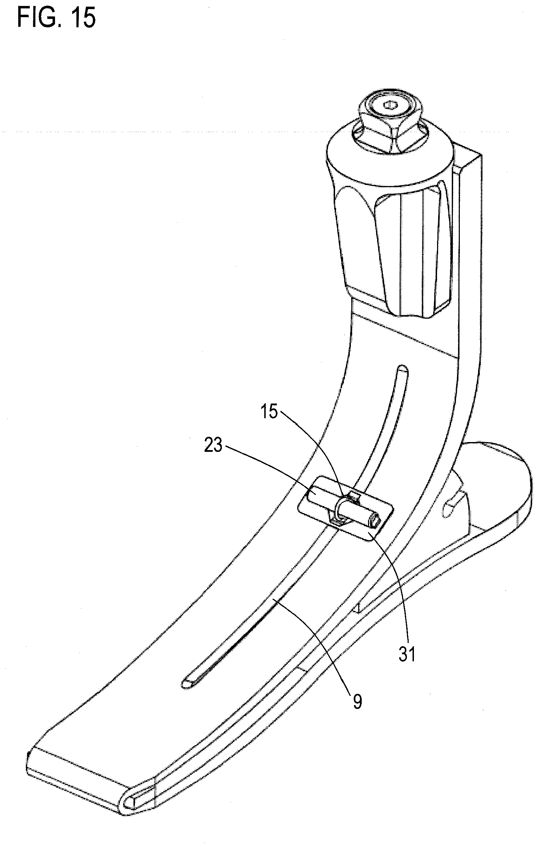

An der Unterseite 35 des Halteblechs 31 sind zwei weitere Laschen 36 vorgesehen, die positionsmäßig mittig zwischen den Laschen 34 angeordnet sind, jedoch wie gesagt zur anderen Seite vorspringen. Die Laschen 36 greifen in den Schlitz 9 ein, siehe die Darstellung gemäß Figur 15. Durch diesen Lascheneingriff ist das Halteblech 31 selbst gegen seitliches Verschieben gesichert, so dass es sich in dieser Richtung nicht bewegen kann.

In der in Figur 15 gezeigten Montagestellung greift die Schlaufe 15 durch den Schlitz 9 sowie die Durchbrechung 32 des Halteblechs 31, der Stift 23 seinerseits durchgreift wiederum die Schlaufe 15. Der Stift selbst ist in der länglichen Durchbrechung 32, die sich, siehe Figur 13, von einer Lasche 34 zur anderen Lasche 34 erstreckt, aufgenommen, kann sich folglich auch in dieser Richtung nicht aus respektive von dem Halteblech 21 bewegen. Das heißt, dass selbst im Falle einer geringfügigen Bewegung längs des Schlitzes 9 stets das Halteblech 31 und der Stift 23 gemeinsam wandern. Ein Lösen des Stiftes 23 vom Halteblech 31 respektive aus der Schlaufe 15 ist damit ausgeschlossen.

## Patentansprüche

1. Fußprothese, umfassend ein Oberteil (2) und ein beim Laufen bodenseitig aufsetzendes Unterteil (3), die übereinander und voneinander beabstandet verlaufen und an der Fußspitze miteinander verbunden sind, wobei das Oberteil (2) und das Unterteil (3) mittels eines einstückigen Bauteils (4) gebildet sind, und wobei im fersennahen Bereich ein elastisches Dämpfungselement (11) zwischen dem Oberteil (2) und dem Unterteil (3) angeordnet und mit dem Oberteil (2) und/oder dem Unterteil (3) verbunden ist, **dadurch gekennzeichnet, dass**
ein das Oberteil (2) und das Unterteil (3) verbindendes, durch eine Ausnehmung (21) im Dämpfungselement (11) und durch einen Drehpunkt, gebildet von dem Dämpfungselement (11) geführtes, druckweiches und zugfestes Verbindungsmittel (13) vorgesehen ist, um welchen Drehpunkt das Oberteil (2) relativ zum Unterteil (3) beim Laufen während des Aufsetzens der Ferse bis zum Abrollen über die Fußspitze drehen kann.

2. Fußprothese nach Anspruch 1, **dadurch gekennzeichnet, dass** das Verbindungsmittel (13) ein Seil (14) oder Band ist.

3. Fußprothese nach Anspruch 2, **dadurch gekennzeichnet, dass** das Seil (14) oder Band aus Kunststofffasern oder Stahldraht ist.

4. Fußprothese nach Anspruch 2 oder 3, **dadurch gekennzeichnet, dass** das Seil (14) oder Band eine Schlaufe (15) bildet, wobei die Enden in einem gemeinsamen Halteelement (16) fixiert sind, das am Unterteil (3) befestigt ist, während die Schlaufe (15) über ein sie durchgreifendes Schlaufenhalteelement (22) am Oberteil (2) festgelegt ist.

5. Fußprothese nach Anspruch 4, **dadurch gekennzeichnet, dass** das Schlaufenhalteelement (22) ein Stift (23) ist, der an der Oberseite (24) des Oberteils (2) aufliegt und wobei der Stift (23) eine das Oberteil (2) in einer Durchbrechung (9) durchgreifenden Schlaufe (15) durchgreift.

6. Fußprothese nach Anspruch 4, **dadurch gekennzeichnet, dass** das Schlaufenhalteelement (22) ein Stift (23) ist, der in eine an der Oberseite (24) des Oberteils (2) angeordneten Halteplatte (26) eingesteckt ist, und wobei der Stift (23) eine das Oberteil (2) in einer Durchbrechung durchgreifenden Schlaufe (15) durchgreift.

7. Fußprothese nach Anspruch 6, **gekennzeichnet durch** ein am Oberteil (2) angeordnetes Halteelement (30), über das der Stift (23) axial gegen ein Verschieben gesichert ist.

8. Fußprothese nach Anspruch 7, **dadurch gekennzeichnet, dass** an dem Halteelement (30), das plättchenförmig ausgeführt ist, eine oder zwei abstehende Laschen (34), die in die oberteilseitige Durchbrechung (9) eingreifen, und zwei weitere Laschen (36), zwischen denen der Stift (23) aufgenommen ist, vorgesehen sind.

9. Fußprothese nach Anspruch 7 oder 8, **dadurch gekennzeichnet, dass** das Halteelement (30) ein Halteblech (31) ist.

10. Fußprothese nach einem der Ansprüche 5 bis 9, **dadurch gekennzeichnet, dass** die Durchbrechung als Schlitz (9), der sich längs des Oberteils (2) erstreckt, oder als Bohrung ausgeführt ist.

11. Fußprothese nach Anspruch 2 oder 3, **dadurch gekennzeichnet, dass** das Seil (2) oder Band mit beiden Enden jeweils in einem Halteelement (16) fixiert ist, wobei das eine Halteelement (16) am Unterteil (3) und das andere Halteelement (16) am Oberteil (2) befestigt ist.

12. Fußprothese nach einem der Ansprüche 4 bis 11, **dadurch gekennzeichnet, dass** ein Halteelement (16) als Hülse (17) ausgeführt ist, in der das Seil (14) oder Band eingeklebt, eingegossen oder eingeklemmt ist, wobei die Hülse (17) das Unterteil (3) oder das Oberteil (2) in einer Durchbrechung (10) durchgreift.

13. Fußprothese nach Anspruch 12, **dadurch gekennzeichnet, dass** die Hülse (17) am Unterteil (3) oder am Oberteil (2) verklebt ist.

14. Fußprothese nach Anspruch 2 oder 3, **dadurch gekennzeichnet, dass** das Seil (14) oder Band mit jeweils einem Ende am Unterteil (3) und am Oberteil (2) einlaminiert ist.

15. Fußprothese nach einem der vorangehenden Ansprüche, **dadurch gekennzeichnet, dass** die am Dämpfungselement (11) vorgesehene Ausnehmung (21) eine Durchgangsbohrung oder Schlitz, der gegebenenfalls an einer Seite des Dämpfungselements (11) offen ist, ist.

16. Fußprothese nach einem der vorangehenden Ansprüche, **dadurch gekennzeichnet, dass** am Dämpfungselement (11) wenigstens ein dessen Dämpfungsverhalten beeinflussendes Versteifungselement anbringbar ist.

17. Fußprothese nach Anspruch 16, **dadurch gekennzeichnet, dass** am Dämpfungselement (11) eine Einsteckaufnahme (12) vorgesehen ist, in die das Versteifungselement einsteckbar ist.

18. Fußprothese nach einem der vorangehenden Ansprüche, **dadurch gekennzeichnet, dass** eine am Oberteil (2) lösbar anbringbare Abdeckung (29), die den Befestigungsbereich des Verbindungsmittels (13) abdeckt, vorgesehen ist.

19. Fußprothese nach einem der vorangehenden Ansprüche, **dadurch gekennzeichnet, dass** das einstückige Bauteil (4) aus einem Kohlenstofffaserverbundwerkstoff ist.

## Claims

1. A foot prosthesis, comprising an upper part (2) and a lower part (3) setting on the ground as the user walks, which extend on top of each other and spaced apart from each other and are connected to each other at the tip of the foot, wherein the upper part (2) and the lower part (3) are formed by means of a one-pieced component (4), and wherein, in the area near the heel, an elastic damping element (11) is arranged between the upper part (2) and the lower part (3) and is connected to the upper part (2) and/or to the lower part (3), **characterized in that**
a compressible but longitudinally strong connecting means (13) which connects the upper part (2) and the lower part (3) and is guided through a recess (21) in the damping element (11) and through a center of rotation, formed by the damping element (11), is provided, around which center of rotation the upper part (2) can rotate relative to the lower part (3), as the user walks, during setting down the heel until rolling off via the tip of the foot.

2. The foot prosthesis according to claim 1, **characterized in that** the connecting means (13) is a cable (14) or strap.

3. The foot prosthesis according to claim 2, **characterized in that** the cable (14) or strap is made of plastic fibers or steel wire.

4. The foot prosthesis according to claim 2 or 3, **characterized in that** the cable (14) or strap forms a loop (15), the ends being held in place in a common retaining element (16) which is attached to the lower part (3), whereas the loop (15) is fixed at the upper part (2) by a loop-retaining element (22) passing through the loop.

5. The foot prosthesis according to claim 4, **characterized in that** the loop-retaining element (22) is a pin (23) which rests on the top surface (24) of the upper part (2), the pin (23) passing through a loop (15) which passes through the upper part (2) in a passage (9).

6. The foot prosthesis according to claim 4, **characterized in that** the loop-retaining element (22) is a pin (23) which is inserted into a retaining plate (26) arranged on the top surface (24) of the upper part (2), and the pin (23) passing through a loop (15) which passes through the upper part (2) in a passage.

7. The foot prosthesis according to claim 6, **characterized by** a retaining element (30) arranged on the upper part (2), by means of which the pin (23) is secured against axial displacement.

8. The foot prosthesis according to claim 7, **characterized in that** on the retaining element (30), which is executed in the form of a plate, one or two projecting tabs (34), which engage in the upper-part-sided passage (9), and two further tabs (36) between which the pin (23) is accommodated are provided.

9. The foot prosthesis according to claim 7 or 8, **characterized in that** the retaining element (30) is a retaining plate (31).

10. The foot prosthesis according to any of claims 5 to 9, **characterized in that** the passage is executed as a slot (9), which extends alongside the upper part (2), or as a hole.

11. The foot prosthesis according to claim 2 or 3, **characterized in that** each of the two ends of the cable (2) or strap is held in place respectively in a retaining element (16), the one retaining element (16) being fastened to the lower part (3) and the other retaining element (16) to the upper part (2).

12. The foot prosthesis according to any of claims 4 to 11, **characterized in that** a retaining element (16) is executed as a sleeve (17), in which the cable (14) or strap is adhesively bonded, cast, or clamped, the sleeve (17) passing through the lower part (3) or the upper part (2) in a passage (10).

13. The foot prosthesis according to claim 12, **characterized in that** the sleeve (17) is adhesively bonded to the lower part (3) or to the upper part (2).

14. The foot prosthesis according to claim 2 or 3, **characterized in that** one end of the cable (14) or strap is laminated into the lower part (3), the other end into the upper part (2).

15. The foot prosthesis according to any of the preceding claims, **characterized in that** the recess (21) provided at the damping element (11) is a through hole or slot which, where applicable, is open at one side of the damping element (11).

16. The foot prosthesis according to any of the preceding claims, **characterized in that** at least one stiffening element can be attached to the damping element (11), which can influence the damping behavior thereof.

17. The foot prosthesis according to claim 16, **characterized in that** a plug-in receptacle (12), into which the stiffening element can be plugged in, is provided on the damping element (11).

18. The foot prosthesis according to any of the preceding claims, **characterized in that** a cover (29) which can be attached detachably on the upper part (2) and which covers the fastening area of the connecting means (13) is provided.

19. The foot prosthesis according to any of the preceding claims, **characterized in that** the one-pieced component (4) is made of a carbon fiber composite material.

## Revendications

1. Prothèse de pied comprenant une partie supérieure (2) et une partie inférieure (3) à poser côté sol lors de la marche, lesquelles sont disposées l'une au-dessus de l'autre et de manière espacée l'une de l'autre et sont reliées entre elles à la pointe du pied, cependant que la partie supérieure (2) et la partie inférieure (3) sont constituées au moyen d'une pièce d'un seul tenant (4), et cependant que, dans la zone proche du talon, un élément élastique d'amortissement (11) est agencé entre la partie supérieure (2) et la partie inférieure (3) et est relié à la partie supérieure (2) et/ou à la partie inférieure (3), **caractérisée en ce que**
un élément de liaison (13) souple à la pression et résistant à la traction est prévu, lequel relie la partie supérieure (2) et la partie inférieure (3) et passe à travers un évidement (21) dans l'élément d'amortissement (11) et à travers un pivot constitué par l'élément d'amortissement (11), pivot autour duquel la partie supérieure (2) peut, relativement à la partie inférieure (3), pivoter lors de la marche pendant que le talon se pose jusqu'au déroulement passant par la pointe du pied.

2. Prothèse de pied selon la revendication 1, **caractérisée en ce que** l'élément de liaison (13) est une corde (14) ou un ruban.

3. Prothèse de pied selon la revendication 2, **caractérisée en ce que** la corde (14) ou le ruban est en fibres en matière plastique ou fil d'acier.

4. Prothèse de pied selon la revendication 2 ou 3, **caractérisée en ce que** la corde (14) ou le ruban constitue une boucle (15), cependant que les extrémités sont attachées dans un élément commun de maintien (16) fixé à la partie inférieure (3), tandis que la boucle (15) est accrochée à la partie supérieure (2) par l'intermédiaire d'un élément de maintien de boucle (22) qui la retient en passant à travers elle.

5. Prothèse de pied selon la revendication 4, **caractérisée en ce que** l'élément de maintien de boucle (22) est une goupille (23) qui s'appuie à la face supérieure (24) de la partie supérieure (2), et cependant que la goupille (23) retient en passant à travers elle une boucle (15) retenant la partie supérieure (2) en passant à travers elle dans un transpercement (9).

6. Prothèse de pied selon la revendication 4, **caractérisée en ce que** l'élément de maintien de boucle (22) est une goupille (23) qui est enfichée dans une plaque de maintien (26) agencée à la face supérieure (24) de la partie supérieure (2), et cependant que la goupille (23) retient en passant à travers elle une boucle (15) retenant la partie supérieure (2) en passant à travers elle dans un transpercement.

7. Prothèse de pied selon la revendication 6, **caractérisée par** un élément de maintien (30) agencé à la partie supérieure (2), par l'intermédiaire duquel la goupille (23) est assurée axialement contre un déplacement.

8. Prothèse de pied selon la revendication 7, **caractérisée en ce que**, à l'élément de maintien (30), qui est réalisé sous forme de plaquette, une ou deux languettes (34) venant en saillie et s'engageant dans le transpercement (9) côté partie supérieure, et deux autres languettes (36) entre lesquelles la goupille (23) est logée sont prévues.

9. Prothèse de pied selon la revendication 7 ou 8, **caractérisée en ce que** l'élément de maintien (30) est une tôle de maintien (31).

10. Prothèse de pied selon une des revendications de 5 à 9, **caractérisée en ce que** le transpercement est réalisé sous forme de fente (9) qui s'étend le long de la partie supérieure (2), ou sous forme d'alésage.

11. Prothèse de pied selon la revendication 2 ou 3, **caractérisée en ce que** la corde (2) ou le ruban est attaché(e) à ses deux extrémités respectivement dans un élément de maintien (16), cependant que le un élément de maintien (16) est fixé à la partie inférieure (3) et l'autre élément de maintien (16) est fixé à la partie supérieure (2).

12. Prothèse de pied selon une des revendications de 4 à 11, **caractérisée en ce qu'**un élément de maintien (16) est réalisé sous forme de douille (17) dans laquelle la corde (14) ou le ruban est collé(e), scellé(e) ou coincé(e), cependant que la douille (17) retient en passant à travers elle la partie inférieure (3) ou la partie supérieure (2) en passant à travers elle dans un transpercement (10).

13. Prothèse de pied selon la revendication 12, **caractérisée en ce que** la douille (17) est collée à la partie inférieure (3) ou à la partie supérieure (2).

14. Prothèse de pied selon la revendication 2 ou 3, **caractérisée en ce que** la corde (14) ou le ruban est laminé(e) par au moins une extrémité à la partie inférieure (3) ou à la partie supérieure (2).

15. Prothèse de pied selon une des revendications précédentes, **caractérisée en ce que** l'évidement (21) prévu dans l'élément d'amortissement (11) est un perçage traversant ou une fente éventuellement ouvert(e) d'un côté de l'élément d'amortissement (11).

16. Prothèse de pied selon une des revendications précédentes, **caractérisée en ce que**, à l'élément d'amortissement (11), au moins un élément de raidissement influençant son comportement d'amortissement peut être monté.

17. Prothèse de pied selon la revendication 16, **caractérisée en ce que**, à l'élément d'amortissement (11), un logement d'enfichage (12) est prévu, dans lequel l'élément de raidissement peut être enfiché.

18. Prothèse de pied selon une des revendications précédentes, **caractérisée en ce qu'**un recouvrement (29) pouvant être placé de manière détachable sur la partie supérieure (2) et recouvrant la zone de fixation de l'élément de liaison (13) est prévu.

19. Prothèse de pied selon une des revendications précédentes, **caractérisée en ce que** la pièce d'un seul tenant (4) est en un matériau composite à fibres de carbone.
